# FASCICULE DE BREVET EUROPEEN

(11) **EP 1 348 456 B1**
(45) Date de publication et mention de la délivrance du brevet: **01.10.2008**
(21) Numéro de dépôt: 03290650.5
(22) Date de dépôt: 14.03.2003
(51) Int. Cl.: A61M 1/02, A61J 1/14, B01D 39/16

(54) **Poche et procédé de défibrination utilisant un élément textile**
Beutel und Defibrinierungsverfahren unter Verwendung eines Textilelements
Bag and defibrination process using a textile element

(30) Priorité: 26.03.2002 FR 0203758
(43) Date de publication de la demande: 01.10.2003
(73) Titulaire: MACO PHARMA, 59420 Mouvaux (FR)
(72) Inventeur: Goudaliez, Francis, 59155 Faches-Thumesnil (FR); Verpoort, Thierry, 59420 Mouvaux (FR)
(74) Mandataire: Geismar, Thierry

(56) Documents cités:
- EP-A- 0 953 361
- WO-A-99/37340
- US-A- 4 073 732
- US-A- 4 129 131
- US-A- 4 437 472
- US-A- 4 701 267
- US-A- 5 616 254

## Description

L'invention concerne une poche destinée à la défibrination d'un fluide biologique, un système à poches comprenant une telle poche, des procédés de défibrination ainsi que l'utilisation d'un élément textile pour la défibrination d'un fluide biologique.

L'invention s'applique typiquement à l'élimination de la fibrine du sang ou du plasma d'un animal ou d'un humain, de sorte à obtenir un sang dit incoagulable ou un sérum sanguin, c'est-à-dire un plasma défibriné.

Lors d'un prélèvement de sang, il est connu d'ajouter des produits chimiques ou anticoagulants au sang afin d'éviter sa coagulation. De tels anticoagulants, contenus dans la poche destinée à recueillir le sang prélevé, sont par exemple du citrate-phosphate-dextrose (CPD) ou de l'acide-citrate-dextrose (ACD).

En effet, de tels anticoagulants permettent, en complexant les ions calcium, de bloquer la cascade de coagulation du sang dès le début de la chaîne. Mais l'ajout d'anticoagulant est parfois indésirable. C'est notamment le cas pour la préparation, à partir de sang incoagulable ou de sérum sanguin, des réactifs biologiques utilisés dans le domaine de la culture cellulaire ou dans le domaine médical.

Une solution pour éviter la coagulation du sang sans ajout d'anticoagulant est la défibrination, c'est-à-dire l'élimination de la fibrine du sang ou du plasma sanguin.

En effet, lors de la coagulation du sang, le fibrinogène, qui est une protéine soluble présente dans le plasma sanguin, se polymérise, sous l'action de la thrombine, en fibrine insoluble. De cette réaction, il résulte la coagulation, c'est-à-dire la formation de caillots sanguins qui comprennent notamment des globules rouges, éventuellement d'autres cellules sanguines et de la fibrine.

Par conséquent, la fibrine est le dernier élément de la cascade de coagulation, et son élimination permet donc d'obtenir un sang incoagulable ou un sérum sanguin.

La défibrination du sang est habituellement obtenue en laissant naturellement le sang coaguler et en séparant le sérum des caillots par décantation et/ou centrifugation.

Mais ce procédé ne permet pas d'obtenir du sang incoagulable. De plus, les caillots formés enferment une quantité non négligeable de sérum, de sorte que le volume de sérum obtenu et donc la quantité de composants utiles à la préparation ultérieure des réactifs sont réduits de façon non négligeable. Enfin, ce procédé est difficilement envisageable dans des cadences de production importantes.

Une autre solution pour la défibrination du sang est le « battage » du sang de sorte à obtenir la fibrine en évitant la formation de caillots. Un tel procédé est par exemple décrit dans le document US-4 129 131 dans lequel des billes rigides ou un matériau réticulé sont utilisés.

Une telle solution présente l'inconvénient, lors de l'agitation, de solliciter fortement le récipient dans lequel les billes ou le matériau réticulé sont placés. C'est pourquoi, pour éviter une possible rupture de l'enveloppe du récipient, elle doit être conduite dans un dispositif rigide de prélèvement, ce qui la rend peu pratique à mettre en oeuvre. En outre, le matériau réticulé présente une capacité de rétention de la fibrine qui est faible, ce qui nécessite la réalisation ultérieure d'une étape de filtration pour séparer la fibrine et le sang.

Le document EP-A1-0 953 361 décrit une poche filtrante renfermant un milieu filtrant contre lequel est disposé un préfiltre et qui délimite deux compartiments. Le pré-filtre a pour fonction d'arrêter, entre autres constituants, la fibrine. Cette poche filtrante est destinée à la déleucocytation du plasma.

Le document WO-A2-99/37340 décrit quant à lui une poche contenant une membrane poreuse apte à retenir au moins un fragment de complément.

Pour pallier ces inconvénients, l'invention propose notamment un procédé de défibrination en une seule étape qui permet d'obtenir de façon simple et efficace un sang incoagulable ou un sérum sanguin, le procédé pouvant être mis en oeuvre dans une structure de poche classiquement utilisée dans le domaine du prélèvement et du traitement du sang.

A cet effet, et selon un premier aspect, l'invention propose une poche destinée à la défibrination d'un fluide biologique tel que le sang ou le plasma sanguin selon la revendication 1.

Selon un deuxième aspect, l'invention propose un système à poches destiné au prélèvement et à la défibrination d'un fluide biologique tel que le sang ou le plasma sanguin selon la revendication 5.

Selon un troisième aspect, l'invention propose un procédé de défibrination d'un fluide biologique tel que du sang ou du plasma sanguin selon la revendication 8.

D'autres objets et avantages de l'invention apparaîtront au cours de la description qui suit, faite en référence aux dessins annexés dans lesquels :
- la figure 1 représente une vue de face d'une poche selon un premier mode de réalisation de l'invention ;
- la figure 2 représente une vue en perspective et en coupe transversale suivant la ligne II-II de la figure 1 ;
- la figure 3 représente une vue de face d'une poche selon un deuxième mode de réalisation de l'invention ;
- la figure 4 représente, en vue schématique de face, un système à poches destiné au prélèvement et à la défibrination du sang.

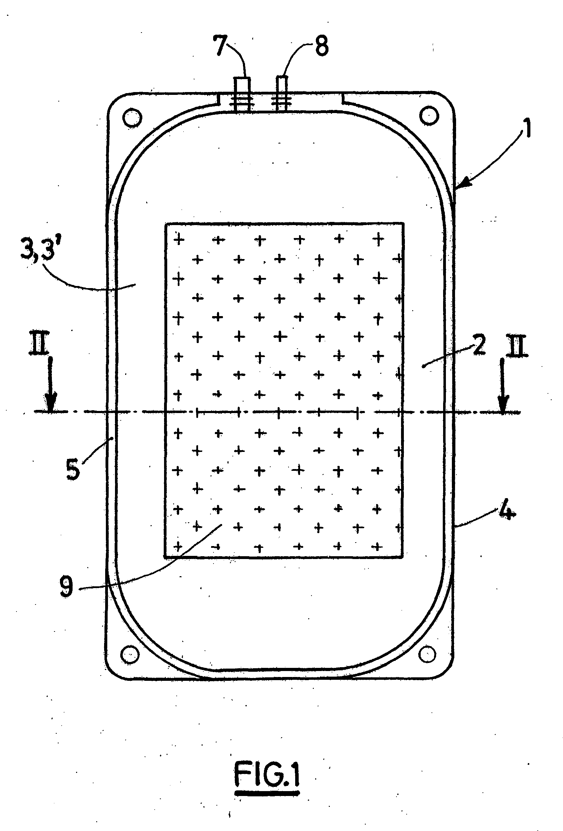

Les figures 1 à 3 représentent une poche 1 destinée à la défibrination du sang ou du plasma sanguin. Dans un exemple particulier, le sang est du sang animal tel que du sang de bovins, ovins, porcins, félins, canins et chevaux. En particulier, le sang est du sang de mouton. Dans un autre exemple particulier, le sang est du sang humain.

Selon l'invention, la poche 1 est souple et comprend une enveloppe extérieure 2 formée de deux feuilles de matériau plastique souple, soudable et stérilisable 3 et 3'. Par exemple, les feuilles 3 et 3' peuvent être en PVC, polyoléfine, éthylène-acétate de vinyle (EVA), polyuréthanne ou polyester.

Ces deux feuilles sont assemblées sur leur périphérie 4, par exemple par un cordon de soudure 5, de sorte à définir un volume intérieur 6 pour le sang.

La poche souple 1 comprend un orifice d'entrée 7 et un orifice de sortie 8 du sang. Ces orifices 7 et 8 sont destinés à être connectés à des tubulures souples, typiquement en matière plastique, destinées à permettre respectivement l'introduction et la récupération du fluide dans la poche 1.

Selon un autre mode de réalisation, non représenté, l'entrée et la sortie du fluide peuvent se faire par un seul et même orifice.

La poche 1 comprend en outre un élément textile 9 disposé dans le volume intérieur 6, sans compartimenter celui-ci.

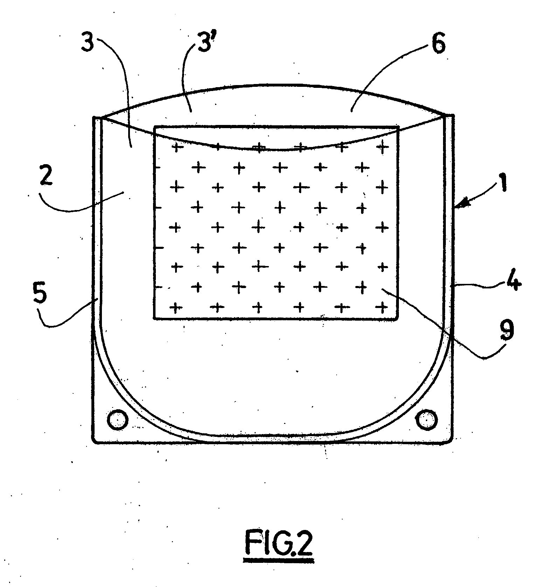

Sur les figures 1 et 2 (premier mode de réalisation), l'élément textile 9 se déplace librement dans la poche 1. Ainsi, lorsque la poche 1 contient le fluide biologique, l'élément textile 9 « flotte» dans la poche 1.

L'élément textile 9 est inséré entre les deux feuilles 3 et 3' lors de la fabrication de la poche 1, avant de réaliser la soudure par exemple.

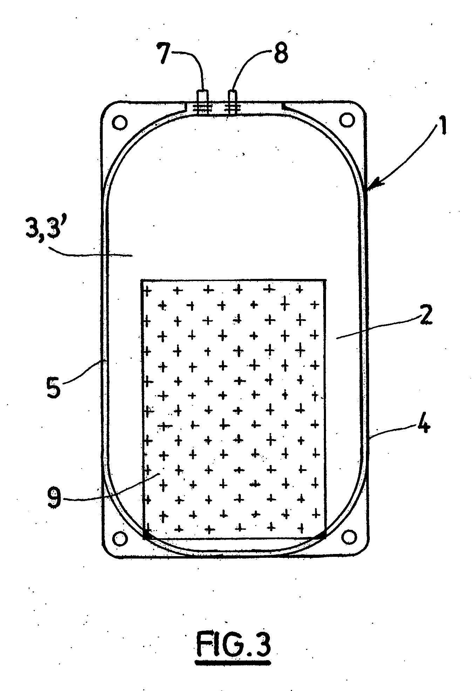

Sur la figure 3 (deuxième mode de réalisation), l'élément textile 9 est fixé localement à l'intérieur de la poche 1. Typiquement, au moins un des bords de l'élément textile 9 est maintenu dans une partie du cordon de soudure 5. Ainsi, l'élément textile 9 est empêché de se replier sur lui-même et/ou de gêner l'écoulement du fluide en bloquant les orifices d'entrée 7 et/ou de sortie 8 de la poche 1.

Dans les modes de réalisation représentés sur les figures 1 à 3, l'élément textile 9 comprend une feuille formée d'au moins une couche de tricot, de tissé ou de non tissé.

Le matériau formant l'élément textile 9 est choisi parmi les polymères ou les copolymères à base de polyéthylène, polypropylène, polyester, polyamide, polyuréthanne et leurs dérivés ou tout autre polymère compatible avec le sang.

L'élément textile 9 est apte à retenir la fibrine. Lors du prélèvement, le fibrinogène se transforme en fibrine sous l'action de la thrombine. Cette fibrine est sous forme de filament. Lorsque les filaments de fibrine entrent en contact avec l'élément textile 9, ils s'enchevêtrent dans les fils et/ou fibres de l'élément 9 et restent ainsi bloqués dans l'élément textile 9. En outre, la porosité et la mouillabilité de l'élément textile 9 permettent d'améliorer la pénétration et la rétention de la fibrine dans celui-ci.

Dans les modes de réalisation représentés, l'élément textile 9 est formé d'une feuille placée à l'intérieur de la poche 1 de sorte que la surface de la feuille est parallèle à la surface des feuilles 3 et 3'. Ainsi, l'épaisseur et le volume de la poche 1 contenant l'élément textile 9 sont sensiblement inchangés par rapport à l'épaisseur et au volume de la même poche sans élément textile 9.

La configuration plane de la feuille assure une surface de contact importante entre la fibrine du sang et l'élément textile 9, et ce sans modifier de façon sensible le volume intérieur 6 de la poche 1.

La surface de l'élément textile 9 est par exemple inférieure à la surface des feuilles 3 et 3' qui forment l'enveloppe 2. Cependant, l'élément textile 9 peut également être plissé ou ondulé, par exemple dans le cas où sa surface est supérieure à celle des feuilles 3 et 3'.

Afin d'augmenter la probabilité de rencontre entre l'élément textile 9 et le fluide, la surface de cet élément représente plus de 50% de la surface des feuilles 3 et 3'.

A titre d'exemple, l'élément textile 9 est constitué d'une feuille rectangulaire de polyester sous forme d'une couche de non-tissé d'une épaisseur d'environ 400 µm et ayant une taille de pore moyenne d'environ 40 µm.

En variante, une autre forme et/ou un nombre différent d'élément textile 9 peut être prévu en fonction notamment de la forme de la poche et/ou de la quantité de fluide à défibriner.

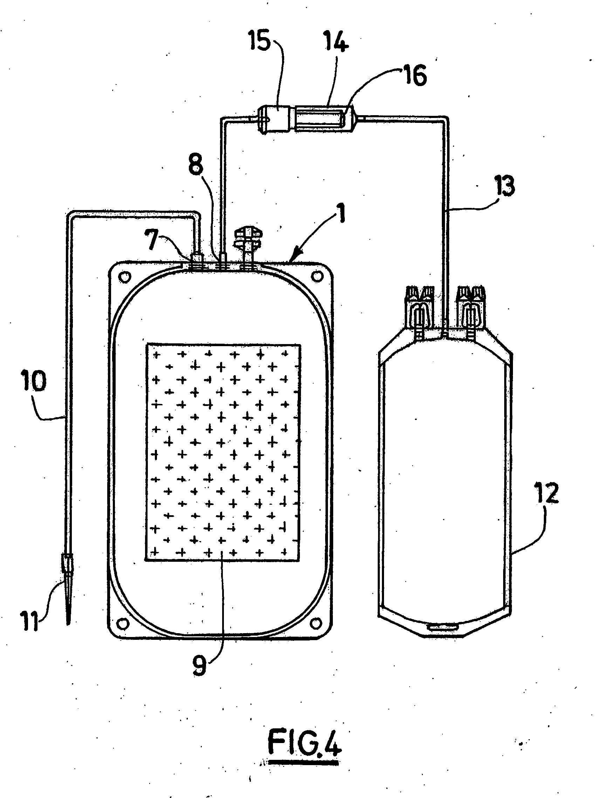

On décrit à présent, en relation avec la figure 4, un mode de réalisation particulier d'un système à poches destiné au prélèvement et à la défibrination du sang en circuit clos, ce système comprenant une poche souple 1 telle que décrite ci-dessus.

La poche 1, dit poche primaire de recueil, est reliée par l'intermédiaire d'une première tubulure 10 et au niveau de l'un des ses orifices d'entrée 7 à des moyens de prélèvement 11. Le système comprend en outre une poche 12, dite poche secondaire, destinée à recevoir le sang défibriné qui est reliée à la poche 1 par l'intermédiaire d'une deuxième tubulure 13 et au niveau de l'un de ses orifices de sortie 8.

La poche secondaire 12 est en matière plastique souple, soudable et stérilisable.

Dans un mode de réalisation particulier, les moyens de prélèvement 11 sont une aiguille de prélèvement.

Le système à poches comprend en outre un filtre capable de retenir les micro-caillots 14 disposé entre la poche primaire 1 et la poche secondaire 12 sur la deuxième tubulure 13. Ce filtre 14 est constitué d'une chambre compte-gouttes 15 en matière plastique comprenant un élément poreux 16 ayant un diamètre de pore d'environ 200 µm, l'élément poreux 16 étant par exemple en Nylon.

Ce filtre 14 permet d'éliminer les derniers filaments de fibrine et les micro-caillots éventuellement présents dans le sang ou le sérum.

Dans un autre mode de réalisation non représenté, le système à poches ne contient pas de filtre capable de retenir les micro-caillots.

Dans ces deux modes de réalisation du système à poches, d'autres poches, dites poches satellites (non représentées) peuvent éventuellement être connectées de façon stérile à la poche primaire 1 et/ou secondaire 12. C'est le cas, par exemple, lorsque le sang défibriné ou le sérum doit subir d'autres traitements tel qu'une centrifugation.

Le système à poches de l'invention est stérilisable et clos, de sorte que le risque d'une contamination bactérienne est limité.

On décrit maintenant deux procédés de prélèvement et de défibrination mettant en oeuvre le système à poches représenté sur la figure 4.

Dans une première étape des deux procédés, on prélève le fluide et on le recueille dans le volume intérieur 6 de la poche 1, ledit volume ne contenant pas de solution anticoagulante et ladite poche 1 étant agitée pendant une durée suffisante pour éliminer sensiblement toute la fibrine du fluide.

Dans la deuxième étape du premier procédé on transfert le sang défibriné dans la poche secondaire 12.

Le transfert du sang défibriné est réalisé via le filtre 14 capable de retenir les micro-caillots.

La poche 12 contenant du sang défibriné peut ensuite être traitée de façon classique par décantation et/ou centrifugation pour préparer du sérum sanguin.

Typiquement, on laisse décanter le sang défibriné contenu dans la poche 12 de façon à obtenir une couche supérieure contenant le sérum et une couche inférieure contenant le sang sans sérum. On sépare ensuite le sérum du sang, puis, éventuellement, on centrifuge à nouveau le sérum de façon à obtenir un surnageant de sérum sensiblement pur, c'est-à-dire dépourvu de composants cellulaires.

En variante, on centrifuge immédiatement le sang défibriné contenu dans la poche secondaire 12 de façon à obtenir une couche supérieure contenant du sérum pratiquement pur et une couche inférieure contenant le sang sans sérum.

Il est alors avantageux d'utiliser un système à poches selon la figure 4 qui comporte en plus au moins une poche satellite en communication fluidique avec la poche secondaire 12 pour recueillir le sérum sanguin.

Dans la deuxième étape du deuxième procédé, après la défibrination du sang, l'agitation de la poche est interrompue de sorte à laisser décanter le sang défibriné dans ladite poche 1 jusqu'à séparation du sérum sanguin et des éléments cellulaires du sang.

Ensuite le sérum est transféré en circuit clos dans la deuxième poche 12.

Le transfert du sérum est réalisé via un filtre 14 capable de retenir les micro-caillots, préférentiellement à l'aide d'une presse.

La poche 12 contient alors du sérum sanguin qui peut éventuellement être centrifugé de façon à obtenir un sérum sensiblement pur acellulaire.

Il est alors avantageux d'utiliser un système à poches selon la figure 4 qui comporte en plus au moins une poche satellite en communication fluidique avec la poche secondaire 12 pour recueillir le sérum sanguin pur.

Pendant l'étape de recueil du sang, on agite la poche 1 afin d'améliorer la mise en contact entre les filaments de fibrine et l'élément textile 9. La poche est en position horizontale et l'agitation peut se faire manuellement ou à l'aide d'un agitateur automatique. Dans un exemple particulier, la poche est posée à plat sur le plateau de l'agitateur automatique en fonctionnement.

Lors de cette agitation, l'élément textile 9, du fait de sa souplesse, ne risque pas d'endommager la poche 1.

Dans un exemple particulier, le rapport du volume de sang prélevé sur le volume total de la poche est inférieur à 1/2, notamment égale à 1/3. Ainsi, la couche de sang contenu dans la poche 1 posée à plat est mince et la surface de contact entre le sang et l'élément textile 9 est étendue.

Selon une réalisation, la deuxième tubulure 13 est sécable et soudable afin de pouvoir dissocier du système à poches la poche secondaire 12 contenant le sang défibriné ou le sérum.

## Revendications

1. Poche (1) destinée à la défibrination d'un fluide biologique tel que le sang ou le plasma sanguin, du type comprenant une enveloppe extérieure (2) formée de deux feuilles (3,3') de matériau plastique souple qui sont assemblées sur leur périphérie, formant un volume intérieur (6) pour ledit fluide, ladite enveloppe (2) étant munie d'au moins un orifice d'entrée (7) et/ou de sortie (8) du fluide, et au moins un élément textile (9) apte à fixer la fibrine du fluide étant disposé dans le volume intérieur (6), sans compartimenter ledit volume intérieur, **caractérisé en ce que** l'élément textile représente plus de 50% de la surface des feuilles (3,3').

2. Poche selon la revendication 1, **caractérisée en ce que** l'élément textile (9) comprend au moins une couche de tricot, de tissé et/ou de non-tissé.

3. Poche selon la revendication 1 ou 2, **caractérisée en ce que** l'élément textile (9) est réalisé à partir d'un polymère ou d'un copolymère à base de polyéthylène, polypropylène, polyester, polyamide, polyuréthanne et leurs dérivés.

4. Poche selon l'une quelconque des revendications 1 à 3, **caractérisée en ce que** l'élément textile (9) est fixé localement sur une partie de la périphérie (4) du volume intérieur (6).

5. Système à poches destiné au prélèvement et à la défibrination d'un fluide biologique tel que le sang ou le plasma sanguin, du type comprenant une poche (1) primaire de recueil du fluide comprenant une enveloppe extérieure (2) formée de deux feuilles (3,3') de matériau plastique souple qui sont assemblées sur leur périphérie, formant un volume intérieur (6) pour ledit fluide, ladite enveloppe (2) étant munie d'au moins un orifice d'entrée (7) auquel des moyens de prélèvement (11) du fluide sont connectés par l'intermédiaire d'une première tubulure (10), et au moins un élément textile (9) apte à fixer la fibrine du fluide est disposé dans le volume intérieur (6), **caractérisé en ce que** l'élément textile représente plus de 50% de la surface des feuilles (3,3').

6. Système selon la revendication 5, **caractérisé en ce qu'**il comprend en outre au moins une deuxième poche (12) de recueil du fluide défibriné qui est reliée à un orifice de sortie (8) de ladite poche (1) par l'intermédiaire d'une deuxième tubulure (13).

7. Système à poches selon la revendication 6, **caractérisé en ce qu'**un filtre (14) apte à retenir les micro-caillots est prévu sur la deuxième tubulure (13).

8. Procédé de défibrination d'un fluide biologique tel que du sang ou du plasma sanguin, dans lequel on recueille le fluide dans le volume intérieur (6) d'une poche (1) selon la revendication 1, ladite poche (1) étant agitée pendant une durée permettant d'éliminer sensiblement toute la fibrine du fluide.

9. Procédé de de défibrination selon la revendication 8, dans lequel ledit volume (6) ne contient pas de solution anticoagulante .

10. Procédé selon la revendication 9, dans lequel le fluide défibriné est ensuite transféré en circuit clos dans une deuxième poche (12) qui est reliée de façon étanche et stérile à la poche (1).

11. Procédé selon la revendication 9, dans lequel le fluide est du sang et, après défibrination du sang, l'agitation de la poche (1) est interrompue de sorte à laisser décanter le sang défibriné dans ladite poche (1) jusqu'à séparation du sérum sanguin et des éléments cellulaires du sang.

12. Procédé selon la revendication 11, dans lequel le sérum est ensuite transféré en circuit clos dans une deuxième poche (12) qui est reliée de façon étanche et stérile à la poche (1).

## Claims

1. A bag (1) intended for the defibrination of a biological fluid such as blood or blood plasma, of the type including an outer covering (2) composed of two sheets (3, 3') made of a flexible plastic material, which are assembled on the periphery thereof, thus forming an interior capacity (6) for said fluid, said covering (2) being provided with at least one fluid inlet (7) and/or outlet (8), and at least one textile element (9) able to fix the fibrin of the fluid being positioned in the interior capacity (6), without partitioning said interior capacity, **characterised in that** the textile element represents over 50% of the surface of the sheets (3, 3').

2. A bag according to claim 1, **characterised in that** the textile element (5) comprises at least a layer of a knitted, woven and/or non-woven material.

3. A bag according to claim 1 or 2, **characterised in that** the textile element (9) is made from a polymer or a copolymer based on polyethylene, polypropylene, polyester, polyamide, polyurethane and the derivatives thereof.

4. A bag according to any one of claims 1 to 3, **characterised in that** the textile element (9) is locally fixed onto a part of the periphery (4) of the interior capacity (6).

5. A bag system intended for the sampling and the defibrination of a biological fluid such as blood or blood plasma, of the type including a primary bag (1) for the collection of the fluid including an outer covering (2) composed of two sheets (3, 3') made of a flexible plastic material, which are assembled on the periphery thereof, thus forming an interior capacity (6) for said fluid, said covering (2) being provided with at least one inlet (7) which the fluid sampling means (11) are connected to, through a first tubing (10) and at least a textile element (9) capable of fixing the fibrin of the fluid being positioned in the interior capacity (6), **characterised in that** the textile element represent over 50% of the surface of the sheets (3, 3').

6. A system according to claim 5, **characterised in that** it further includes at least a second bag (12) for the collection of the defibrinated fluid, which is connected to an outlet (8) of said bag (1) through a second tubing (13).

7. A bag system according to claim 6, **characterised in that** one filter (14) able to hold the micro-clots is provided on the second tubing (13).

8. A method for defibrinating a biologic fluid such as blood or blood plasma, wherein the fluid is collected in the interior capacity (6) of the bag (1) according to claim 1, said bag (1) being stirred for a time making it possible to eliminate substantially the whole fibrin in the fluid.

9. Defibrination method according to claim 8, in which said volume (6) contains no anti-coagulant solution.

10. A method according to claim 9, wherein the defibrinated fluid is then transferred in a closed circuit to a second bag (12) which is connected to the bag (1) in a sealing and sterile way.

11. A method according to claim 9, wherein the fluid is blood and upon completion of the blood defibrination, the stirring of the bag (1) is interrupted so as to let the defibrinated blood settle down in said bag (1) until the blood serum and the blood cell elements are separated.

12. A method according to claim 11, wherein the serum is then transferred in closed circuit to a second bag (12) which is connected to the bag (1) in a sealing and sterile way.

## Patentansprüche

1. Beutel (1) für die Defibrination eines biologischen Mediums wie Blut oder Blutplasma, bestehend aus einem Außenmantel (2), der aus zwei Folien (3, 3') aus flexiblem Kunststoff gebildet wird, die an ihrem umfang zusammengefügt werden und damit ein Innenvolumen (6) für das genannte Medium bilden, wobei der genannte Mantel (2) mindestens eine Eingangsöffnung (7) und/oder Ausgangsöffnung (8) für das Medium aufweist, und wobei mindestens ein Textilelement (9), das das Fibrin des Mediums fixieren kann, in dem Innenvolumen (6) angeordnet ist, ohne das genannte Innenvolumen zu unterteilen, **dadurch gekennzeichnet, dass** das Textilelement über 50% der Oberfläche der Folien (3, 3') darstellt.

2. Beutel gemäß Anspruch 1, **dadurch gekennzeichnet, dass** das Textilelement (9) mindestens eine aus Strickgewebe, Webstoff und/oder Vliesstoff bestehende Schicht aufweist.

3. Beutel gemäß Anspruch 1 oder 2, **dadurch gekennzeichnet, dass** das Textilelement (9) aus einem Polymer oder einem Copolymer auf Basis von Polyethylen, Polypropylen, Polyester, Polyamid, Polyurethan oder ihren Derivaten hergestellt wird.

4. Beutel gemäß einem der Ansprüche 1 bis 3, **dadurch gekennzeichnet, dass** das Textilelement (9) lokal an einem Teil des Umfangs (4) des Innenvolumens (6) befestigt ist.

5. Beutelsystem für die Entnahme und die Defibrination eines biologischen Mediums wie Blut oder Blutplasma, bestehend aus einem Primärsammelbeutel (1) des Mediums, mit einem Außenmantel (2), der aus zwei Folien (3, 3') aus flexiblem Kunststoff gebildet wird, die an ihrem Rand zusammengefügt werden und damit ein Innenvolumen (6) für das genannte Medium bilden, wobei der genannte Mantel (2) mindestens eine Eingangsöffnung (7) aufweist, an der die Entnahmemittel (11) des Mediums über einen ersten Stutzen (10) angeschlossen sind, und mindestens ein Textilelement (9), das das Fibrin des Mediums fixieren kann, in dem Innenvolumen (6) angeordnet ist, **dadurch gekennzeichnet, dass** das Textilelement über 50% der Oberfläche der Folien (3, 3') darstellt.

6. System gemäß Anspruch 5, **dadurch gekennzeichnet, dass** es ferner mindestens einen zweiten Sammelbeutel (12) des defibrinierten Mediums umfasst, der über einen zweiten Stutzen (13) an eine Ausgangsöffnung (8) des genannten Beutels (1) angeschlossen ist.

7. Beutelsystem gemäß Anspruch 6, **dadurch gekennzeichnet, dass** an dem zweiten Stutzen (13) ein Filter (14) vorgesehen ist, der die Mikroklumpen zurückhalten kann.

8. Defibrinationsverfahren eines biologischen Mediums wie Blut oder Blutplasma, bei dem man das Medium im Innenvolumen (6) eines Beutels (1) gemäß Anspruch 1 sammelt, wobei der genannte Beutel (1) während einer Dauer bewegt wird, die das Beseitigen etwa des gesamten Fibrins aus dem Medium ermöglicht.

9. Defibrinationsverfahren gemäß Anspruch 8, bei dem das genannte Volumen (6) keine koagulationshemmende Lösung enthält.

10. Verfahren gemäß Anspruch 9, bei dem das defibrinierte Medium dann im geschlossenen Kreislauf in einen zweiten Beutel (12) transferiert wird, der dicht und steril mit dem Beutel (1) verbunden ist.

11. Verfahren gemäß Anspruch 9, bei dem das Medium Blut ist, und bei dem nach der Defibrination des Bluts die Bewegung des Beutels (1) unterbrochen wird, so dass das defibrinierte Blut in dem genannten Beutel (1) bis zur Trennung des Blutserums und der Zellenelemente des Bluts dekantiert wird.

12. Verfahren gemäß Anspruch 11, bei dem das Serum dann in geschlossenem Kreislauf in einen zweiten Beutel (12) transferiert wird, der dicht und steril mit dem Beutel (1) verbunden ist.
